(19) Europäisches Patentamt
European Patent Office
Office européen des brevets

(11) Veröffentlichungsnummer: 0 023 308
B1

(12) # EUROPÄISCHE PATENTSCHRIFT

(45) Veröffentlichungstag der Patentschrift: 22.12.82

(51) Int. Cl.[3]: C 07 D 211/44, C 07 D 491/113, A 01 N 25/32

(21) Anmeldenummer: 80104064.3

(22) Anmeldetag: 14.07.80

(54) **N-Acyl-piperidonketale, Verfahren zu deren Herstellung und deren Verwendung als Gegenmittel zum Schutz von Kulturpflanzen vor Schädigungen durch Herbizide.**

(30) Priorität: 26.07.79 DE 2930449

(43) Veröffentlichungstag der Anmeldung: 04.02.81 Patentblatt 81/5

(45) Bekanntmachung des Hinweises auf die Patenterteilung: 22.12.82 Patentblatt 82/51

(84) Benannte Vertragsstaaten: BE CH DE FR GB IT LI NL

(56) Entgegenhaltungen:
EP-A-0 008 649
FR-A-2 133 793
FR-A-2 168 848
FR-A-2 324 637
FR-A-2 392 007
US-A-4 188 485

„Chemical Abstracts", Bd. 83, 1975, Zusammenfassung Nr. 28237v, S. 512, Columbus, Ohio, US
„Chemical Abstracts", Bd. 82, 1975, Zusammenfassung Nr. 140108j, S. 627, Columbus, Ohio, US
„Journal of Agricultural and Food Chemistry", Bd. 26, Nr. 1, 1978, Washington, DC, US, G. R. STEPHENSON *et al.*: „Structure activity relationships for S-ethyl N,N-dipropylthiocarbamate (EPTC) antidotes in Corn", S. 137-140

(73) Patentinhaber: BAYER AG, Zentralbereich Patente, Marken und Lizenzen, D-5090 Leverkusen 1, Bayerwerk (DE)

(72) Erfinder: Riebel, Hans-Jochem, Dr., In der Beek 92, D-5600 Wuppertal 1 (DE)
Erfinder: Eue, Ludwig, Dr., Paul-Klee-Strasse 36, D-5090 Leverkusen 1 (DE)
Erfinder: Faust, Wilfried, Dr., Eifgenstrasse 16, D-5068 Odenthal 3 (DE)
Erfinder: Priesnitz, Uwe Dr., Heinrich-Heine-Strasse 42, D-4750 Unna-Massen (DE)

**Die Akte enthält technische Angaben, die nach dem Eingang der Anmeldung eingereicht wurden und die nicht in dieser Patentschrift enthalten sind.**

EP 0 023 308 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# N-Acylpiperidonketale, Verfahren zu deren Herstellung und deren Verwendung als Gegenmittel zum Schutz von Kulturpflanzen vor Schädigungen durch Herbizide

Die vorliegende Erfindung betrifft neue N-Acylpiperidonketale, mehrere Verfahren zu deren Herstellung sowie deren Verwendung als Gegenmittel zum Schutz von Kulturpflanzen vor Schädigungen durch herbizid wirksame Thiolcarbamate und Acetanilide. Ferner betrifft die vorliegende Erfindung neue Wirkstoffkombinationen, die aus neuen N-Acylpiperidonketalen und bestimmten herbizid wirksamen Thiolcarbamaten bzw. Acetaniliden bestehen, und besonders gute selektive herbizide Eigenschaften besitzen.

Unter Gegenmitteln (Safener, Antidote) sind im vorliegenden Zusammenhang Stoffe zu verstehen, welche befähigt sind, schädigende Wirkungen von Herbiziden auf Kulturpflanzen spezifisch zu antagonisieren, d.h. die Kulturpflanzen zu schützen, ohne dabei die Herbizidwirkung auf die zu bekämpfenden Unkräuter merklich zu beeinflussen.

Es ist bekannt, dass bestimmte Thiolcarbamate und Acetanilide beim Einsatz zur Unkrautbekämpfung in Mais und anderen Kulturen mehr oder weniger starke Schäden an den Kulturpflanzen hervorrufen. Weiterhin ist bekannt, dass Verbindungen wie z.B. N-Dichloracetyl-2-ethylpiperidin und N-Dichloracetyl-cis,trans-decahydrochinolin geeignet sind, um Schädigungen durch Thiolcarbamate oder Acetanilide an Kulturpflanzen zu vermindern (vgl. DE-OS Nr. 2218097). Die Wirksamkeit dieser Stoffe als Gegenmittel ist jedoch nicht immer ganz befriedigend.

Es wurden jetzt neue N-Acylpiperidonketale der Formel gefunden

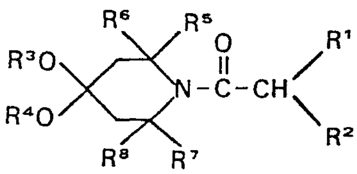

(I)

in welcher

$R^1$ für Alkyl oder Halogen steht,

$R^2$ für Halogen steht,

$R^3$ für Alkyl steht,

$R^4$ für Alkyl steht, oder

$R^3$ und $R^4$ gemeinsam für eine gegebenenfalls verzweigte Alkylenkette stehen, und

$R^5$, $R^6$, $R^7$ und $R^8$ unabhängig voneinander für Wasserstoff oder Alkyl stehen.

Weiterhin wurde gefunden, dass sich N-Acylpiperidonketale der Formel I herstellen lassen, wenn man

a) Piperidonketale der Formel

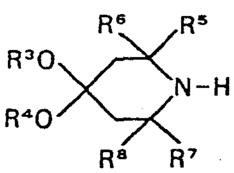

(II)

in welcher

$R^3$, $R^4$, $R^5$, $R^6$, $R^7$ und $R^8$ die oben angegebene Bedeutung haben,

mit Alkanoylchloriden der Formel

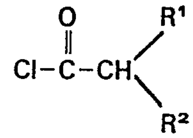

(III)

in welcher

$R^1$ und $R^2$ die oben angegebene Bedeutung haben,

gegebenenfalls in Gegenwart eines Verdünnungsmittels sowie gegebenenfalls in Gegenwart eines Säurebindemittels umsetzt,

oder

b) N-Acylpiperidone der Formel

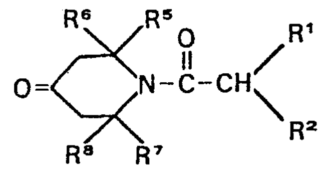

(IV)

in welcher

$R^1$, $R^2$, $R^5$, $R^6$, $R^7$ und $R^8$ die oben angegebene Bedeutung haben,

mit Alkoholen der Formel

$$R^9{-}OH \qquad (V)$$

in welcher

$R^9$ für Alkyl oder ω-Hydroxyalkyl steht,

in Gegenwart eines Katalysators oder eines Wasserbindemittels sowie gegebenenfalls in Gegenwart eines Verdünnungsmittels umsetzt.

Ferner wurde gefunden, dass N-Acylpiperidonketale der Formel I sich hervorragend verwenden lassen, um Kulturpflanzen vor Schädigungen durch herbizid wirksame Thiolcarbamate bzw. durch herbizid wirksame Acetanilide zu schützen.

Ausserdem wurde gefunden, dass die neuen Wirkstoffkombinationen, bestehend aus einem N-Acylpiperidonketal der Formel I und mindestens einem herbizid wirksamen Thiolcarbamat bzw. mindestens einem herbizid wirksamen Acetanilid hervorragend geeignet sind zur selektiven Unkrautbekämpfung in Nutzpflanzenkulturen.

Überraschenderweise werden Herbizidschädigungen durch Thiolcarbamate bzw. durch Acetanilide an Kulturpflanzen bei Mitverwendung von N-Acylpiperidonketalen der Formel I besser unterdrückt als beim Einsatz der bekannten Verbindungen N-Dichloracetyl-2-ethylpiperidin und N-Dichloracetyl-cis,trans-decahydrochinolin, welches chemisch ähnliche Stoffe gleicher Wirkungsart sind. Im übrigen war nicht zu erwarten, dass die erfindungsgemässen Wirkstoffkombinationen bessere selektive herbizide Eigenschaften besitzen als Wirkstoffkombinationen, welche aus mindestens einem herbizid wirksamen Thiolcarbamat bzw. mindestens einem herbizid wirksamen Acetanilid und dem als Gegenmittel bekannten N-Dichloracetyl-2-ethylpiperidin oder dem ebenfalls

als Gegenmittel bekannten N-Dichloracetyl-cis, trans-decahydrochinolin bestehen.

Die erfindungsgemässen N-Acylpiperidonketale sind durch die Formel I allgemein definiert. In dieser Formel steht $R^1$ vorzugsweise für geradkettiges oder verzweigtes Alkyl mit 1 bis 4 Kohlenstoffatomen sowie für Chlor oder Brom. $R^2$ steht vorzugsweise für Chlor oder Brom. $R^3$ steht vorzugsweise für geradkettiges oder verzweigtes Alkyl mit 1 bis 4 Kohlenstoffatomen. $R^4$ steht ebenfalls vorzugsweise für geradkettiges oder verzweigtes Alkyl mit 1 bis 4 Kohlenstoffatomen. Ferner stehen $R^3$ und $R^4$ gemeinsam vorzugsweise für eine gegebenenfalls durch Methyl und/oder Ethyl substituierte Alkylenkette mit 2 oder 3 Kohlenstoffatomen. Die Reste $R^5$, $R^6$, $R^7$ und $R^8$ stehen unabhängig voneinander vorzugsweise für Wasserstoff, Methyl oder Ethyl.

Verwendet man 4-Glykolketalpiperidon und α-Chlorpropionsäurechlorid als Ausgangsstoffe, so lässt sich der Verlauf des erfindungsgemässen Verfahrens a durch das folgende Formelschema wiedergeben:

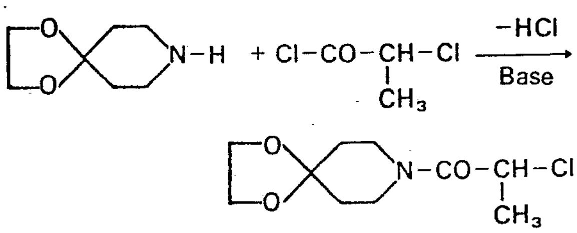


Verwendet man N-Dichloracetyl-4-piperidon und Methanol als Ausgangsstoffe, so lässt sich der Verlauf des erfindungsgemässen Verfahrens b durch das folgende Formelschema wiedergeben:

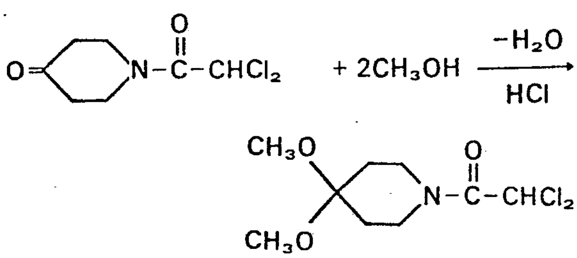


Die bei dem erfindungsgemässen Verfahren a als Ausgangsstoffe benötigten Piperidonketale sind durch die Formel II allgemein definiert. In dieser Formel stehen $R^3$, $R^4$, $R^5$, $R^6$, $R^7$ und $R^8$ vorzugsweise für diejenigen Reste, die bereits im Zusammenhang mit der Beschreibung der erfindungsgemässen Verbindungen der Formel I vorzugsweise für diese Reste genannt wurden.

Die Piperidonketale der Formel II sind teilweise bekannt [vgl. BE-PS Nr. 660763 und „Experientia" *20*, S. 437-438 (1964)]. Die bisher noch nicht bekannten Piperidonketale der Formel II lassen sich in einfacher Weise nach im Prinzip bekannten Methoden herstellen. So erhält man Piperidonketale der Formel II, wenn man die entsprechenden 4-Piperidone mit den jeweiligen Alkoholen in Gegenwart eines sauren Katalysators, wie z.B. Chlorwasserstoff, bei Temperaturen zwischen 0 und 50° C umsetzt.

Die bei dem erfindungsgemässen Verfahren a weiterhin als Ausgangsstoffe benötigten Alkanoylchloride sind durch die Formel III allgemein definiert. In dieser Formel haben $R^1$ und $R^2$ vorzugsweise diejenigen Bedeutungen, die bereits im Zusammenhang mit der Beschreibung der erfindungsgemässen Verbindungen der Formel I vorzugsweise für $R^1$ und $R^2$ genannt wurden.

Die Alkanoylchloride der Formel III sind bekannt oder lassen sich nach im Prinzip bekannten Verfahren in einfacher Weise herstellen (vgl. DE-OS Nr. 2218097).

Das erfindungsgemässe Verfahren a wird vorzugsweise in Gegenwart eines Verdünnungsmittels durchgeführt. Als Verdünnungsmittel können hierbei Wasser sowie inerte organische Lösungsmittel verwendet werden. Hierzu gehören vorzugsweise Ketone, wie Diethylketon und Methylisobutylketon; Nitrile, wie Propionitril und Acetonitril; Ether, wie Tetrahydrofuran oder Dioxan; aliphatische und aromatische Kohlenwasserstoffe, wie Petrolether, Benzol, Toluol und Xylol, halogenierte Kohlenwasserstoffe, wie Methylenchlorid, Tetrachlorkohlenstoff, Chloroform oder Chlorbenzol; Ester, wie Essigester; und Formamide, wie insbesondere Dimethylformamid.

Bei der Durchführung des erfindungsgemässen Verfahrens a kommen als Säurebindemittel alle üblichen Säureakzeptoren in Betracht. Hierzu gehören vorzugsweise Alkalihydroxide, wie Natriumhydroxid und Kaliumhydroxid, ferner Alkalicarbonate, wie Natriumcarbonat, Kaliumcarbonat und Natriumhydrogencarbonat, und weiterhin niedere tertiäre Amine, wie Triethylamin, Dimethylbenzylamin, Pyridin, Diazabicyclooctan und 1,8-Diazabicyclo-[5.4.0]-undec-7-en. Es kann jedoch auch im Überschuss eingesetztes Piperidonketal der Formel II gleichzeitig als Säurebindemittel fungieren. In diesem Fall erübrigt sich die Zugabe eines zusätzlichen Säurebindemittels.

Die Reaktionstemperaturen können beim erfindungsgemässen Verfahren a in einem grösseren Bereich variiert werden. Im allgemeinen arbeitet man zwischen etwa 0 und 100° C, vorzugsweise zwischen 10 und 80° C.

Bei der Durchführung des erfindungsgemässen Verfahrens a setzt man auf 1 mol an Piperidonketal der Formel II vorzugsweise 1 bis 2 mol Alkanoylchlorid der Formel III sowie gegebenenfalls 1 mol Säurebindemittel ein. Die Isolierung der Reaktionsprodukte erfolgt nach üblichen Methoden. Im allgemeinen verfährt man in der Weise, dass man das Reaktionsgemisch nach beendeter Umsetzung filtriert, das Filtrat einengt und den verbleibenden Rückstand gegebenenfalls umkristallisiert.

Die bei dem erfindungsgemässen Verfahren b als Ausgangsstoffe benötigten N-Acylpiperidone sind durch die Formel IV allgemein definiert. In dieser Formel haben $R^1$, $R^2$, $R^5$, $R^6$, $R^7$ und $R^8$ vorzugsweise diejenigen Bedeutungen, die bereits im Zusammenhang mit der Beschreibung der erfindungsgemässen Verbindungen der Formel I vorzugsweise für diese Reste genannt wurden.

Die N-Acylpiperidone der Formel IV sind bisher

noch nicht bekannt. Sie lassen sich jedoch in einfacher Weise nach im Prinzip bekannten Verfahren herstellen. So erhält man N-Acylpiperidone der Formel IV, wenn man Piperidone der Formel

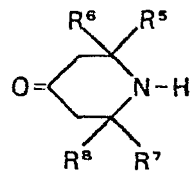

(VI)

in welcher

$R^5$, $R^6$, $R^7$ und $R^8$ die oben angegebene Bedeutung haben,

mit Alkanoylchloriden der Formel

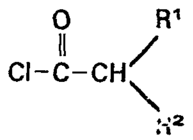

(III)

in welcher

$R^1$ und $R^2$ die oben angegebene Bedeutung haben,

gegebenenfalls in Gegenwart eines Verdünnungsmittels sowie gegebenenfalls in Gegenwart eines Säurebindemittels umsetzt.

Als Verdünnungsmittel und Säurebindemittel kommen hierbei diejenigen Solventien und Säureakzeptoren in Betracht, die bereits im Zusammenhang mit dem erfindungsgemässen Verfahren a genannt wurden. Auch die Reaktionstemperaturen und die übrigen Umsetzungsbedingungen entsprechen denjenigen des Verfahrens a. Die Piperidone der Formel VI sind bekannt oder lassen sich nach im Prinzip bekannten Verfahren herstellen.

Die bei dem erfindungsgemässen Verfahren b weiterhin als Ausgangsstoffe benötigten Alkohole sind durch die Formel V allgemein definiert. In dieser Formel steht $R^9$ vorzugsweise für geradkettiges oder verzweigtes Alkyl mit 1 bis 4 Kohlenstoffatomen sowie für geradkettiges oder verzweigtes ω-Hydroxyalkyl mit 2 bis 7 Kohlenstoffatomen.

Als Beispiele für Alkohole der Formel V seien im einzelnen genannt:

Methanol, Ethanol, Propanol, Ethylenglykol, Propandiol-1,3.

Die Alkohole der Formel V sind bekannt.

Als Katalysatoren können bei der Durchführung des erfindungsgemässen Verfahrens b alle üblicherweise für Ketalisierungen verwendeten Verbindungen eingesetzt werden. Hierzu gehören vorzugsweise Säuren, wie Chlorwasserstoff, Bromwasserstoff und Toluolsulfonsäure.

Die Ketalisierung kann auch in Gegenwart eines Wasserbindemittels durchgeführt werden. Als solches sei Trimethylchlorsilan genannt.

Als Verdünnungsmittel kommen bei der Durchführung des erfindungsgemässen Verfahrens b vorzugsweise die als Reaktionskomponenten fungierenden Alkohole in Frage. Es ist jedoch auch möglich, andere inerte organische Solventien einzusetzen. Vorzugsweise in Frage kommen hierbei aliphatische oder aromatische, gegebenenfalls halogenierte Kohlenwasserstoffe, wie z.B. Benzol, Toluol, Methylenchlorid, Chloroform oder Tetrachlorkohlenstoff.

Die Reaktionstemperaturen können bei der Durchführung des erfindungsgemässen Verfahrens b innerhalb eines grösseren Bereichs variiert werden. Im allgemeinen arbeitet man bei Temperaturen zwischen 0 und 200° C, vorzugsweise zwischen 20 und 150° C.

Bei der Durchführung des erfindungsgemässen Verfahrens b setzt man auf 1 mol N-Acylpiperidon der Formel IV 2 bis 3 mol oder auch einen grösseren Überschuss an Alkohol der Formel V sowie eine geringe Menge an Katalysator oder einen Überschuss an Wasserbindemittel ein. Die Isolierung der Reaktionsprodukte erfolgt nach üblichen Methoden. Im allgemeinen verfährt man in der Weise, dass man die flüchtigen Komponenten sorgfältig abdestilliert und gegebenenfalls den Rückstand umkristallisiert.

Die erfindungsgemässen N-Acylpiperidonketale der Formel I eignen sich — wie bereits erwähnt —, zum Schutz von Kulturpflanzen vor Schädigungen durch herbizid wirksame Thiolcarbamate und Acetanilide, ohne deren Unkrautwirkung merklich zu beeinflussen.

Vorzugsweise können die N-Acylpiperidonketale der Formel I als Gegenmittel zum Schutz von Kulturpflanzen vor Schädigungen durch herbizid wirksame Thiolcarbamate der Formel

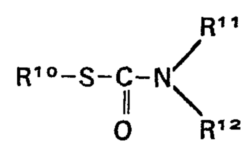

(VII)

in welcher

$R^{10}$ für niederes Alkyl, Benzyl, Chlorbenzyl oder Alkoxybenzyl steht,

$R^{11}$ und $R^{12}$ unabhängig voneinander für Alkyl mit 2 bis 4 Kohlenstoffatomen oder für Cyclohexyl stehen, und ausserdem,

$R^{11}$ und $R^{12}$ gemeinsam mit dem angrenzenden Stickstoffatom für einen fünf- bis siebengliedrigen heterocyclischen Ring stehen,

bzw. zum Schutz von Kulturpflanzen vor Schädigungen durch herbizid wirksame Acetanilide — der Formel

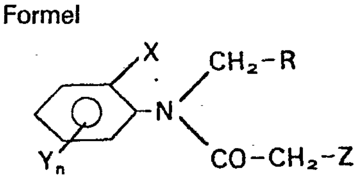

(VIII)

in welcher

R für einen gegebenenfalls substituierten N-haltigen heterocyclischen Rest steht,

X und Y gleich oder verschieden sind und für Alkyl stehen,

Z für Halogen steht, und

n für 0, 1 oder 2 steht,

sowie deren herbizid wirksame Säureadditionssalze und Metallsalzkomplexe,

— bzw. der Formel

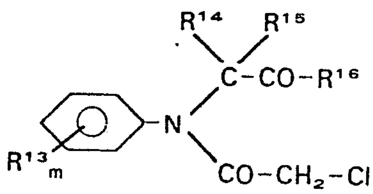

(IX)

in welcher

$R^{13}$ für Alkyl, Halogen, Halogenalkyl, Alkylthio, Alkylsulfonyl, Aminosulfonyl, Cyano oder Nitro steht,

$R^{14}$ und $R^{15}$ gleich oder verschieden sind und für Wasserstoff, Alkyl, Halogen, Halogenalkyl oder gegebenenfalls substituiertes Phenyl stehen,

$R^{16}$ für Alkyl oder gegebenenfalls substituiertes Phenyl steht, und

m für ganze Zahlen von 0 bis 5 steht,

— bzw. der Formel

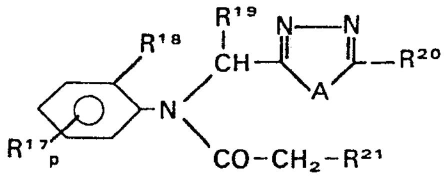

(X)

in welcher

A für Sauerstoff, Schwefel oder die Gruppierung $\rangle NR^{22}$ steht,

$R^{19}$ für Wasserstoff oder Alkyl steht,

$R^{20}$ für Wasserstoff, Alkyl, Halogenalkyl, Alkenyl, Alkinyl, Cycloalkyl, Halogen, gegebenenfalls substituiertes Aryl und Aralkyl oder die Gruppierungen $-OR^{23}$, $-SR^{23}$ und $NR^{22}R^{23}$ steht,

$R^{22}$ für Wasserstoff, Alkyl oder gegebenenfalls substituiertes Aryl steht,

$R^{23}$ für Wasserstoff, Alkyl, Halogenalkyl, Alkenyl, Alkinyl, Cycloalkyl oder gegebenenfalls substituiertes Aralkyl steht,

$R^{17}$ für Alkyl steht,

$R^{18}$ für Alkyl oder Halogen steht,

$R^{21}$ für Halogen steht, und

P für die Zahlen 0, 1 oder 2 steht,

— bzw. der Formel

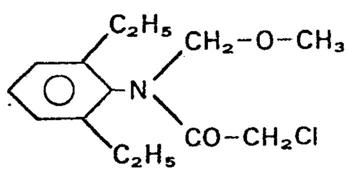

(XI)

— oder der Formel

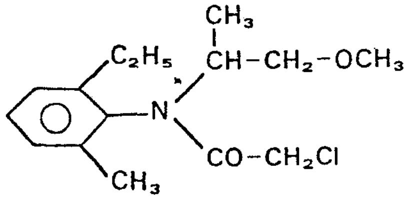

(XII)

verwendet werden.

Als Beispiele für Thiolcarbamate der Formel VII seien im einzelnen genannt:

S-Ethyl-N,N-dipropylthiocarbamat
S-Ethyl-N,N-diisobutylthiocarbamat
S-Propyl-N-butyl-N-ethylthiocarbamat
S-Propyl-N,N-diisopropylthiocarbamat
S-Ethyl-N,N-diethylthiocarbamat
S-Ethyl-N-ethyl-N-cyclohexylthiocarbamat
S-Ethylhexahydroazepin-1-thiocarbamat
S-p-Methoxybenzyl-N,N-diethylthiocarbamat
S-p-Chlorbenzyl-N,N-diethylthiocarbamat
S-Benzyl-N,N-diethylthiocarbamat
S-Benzyl-N,N-di-sek.-butylthiocarbamat
S-Propyl-N-ethyl-N-butylthiocarbamat

Die Thiolcarbamate der Formel VII und deren herbizide Wirksamkeit sind bereits bekannt (vgl. US-Patentschriften Nrn. 2913327, 3037853, 3185720, 3198786 und 3582314).

In der Formel VIII steht R vorzugsweise für die gegebenenfalls substituierten Azolylreste Pyrazol-1-yl, Imidazol-1-yl, 1,2,4-Triazol-1-yl; 1,2,3-Triazol-1-yl; 1,3,4-Triazol-1-yl und 1,2,3,4-Tetrazol-1-yl sowie für gegebenenfalls substituiertes Pyrrol-1-yl. Als Substituenten kommen vorzugsweise in Frage: Halogen, insbesondere Fluor, Chlor und Brom sowie Alkyl mit 1 bis 4 Kohlenstoffatomen. X und Y sind gleich oder verschieden und stehen vorzugsweise für geradkettiges oder verzweigtes Alkyl mit 1 bis 4 Kohlenstoffatomen. Z steht vorzugsweise für die Halogene Chlor und Brom und der Index n steht für 0, 1 oder 2.

Als Beispiele für Acetanilide der Formel VIII seien im einzelnen genannt:

2-Methyl-6-ethyl-N-(pyrazol-1-yl-methyl)-chloracetanilid

2,6-Diethyl-N-(pyrazol-1-yl-methyl)chloracetanilid

2,6-Diethyl-N-(1,2,4-triazol-1-yl-methyl)-chloracetanilid

2,6-Dimethyl-N-(1,2,4-triazol-1-yl-methyl)-chloracetanilid

2-Methyl-N-(pyrazol-1-yl-methyl)chloracetanilid

2,5-Dimethyl-N-(pyrazol-1-yl-methyl)chloracetanilid

2,3-Dimethyl-N-(pyrazol-1-yl-methyl)chloracetanilid

2-Methyl-6-ethyl-N-(pyrazol-1-yl-methyl)-chloracetanilidhydrochlorid

2,6-Diethyl-N-(pyrazol-1-yl-methyl)chloracetanilidhydrochlorid

2,6-Diethyl-N[(3,5-dimethylpyrazol-1-yl)methyl]chloracetanilid

2,6-Dietyl-N-[(3-chlor-1,2,4-triazolyl)methyl]chloracetanilid

2-Methyl-6-ethyl-N-[(3,5-dimethylpyrazol-1-yl)methyl]chloracetanilid

2-tert.-Butyl-N-(pyrazol-1-yl-methyl)chloracetanilid

2-Methyl-6-ethyl-N-[(3-brom-5-methylpyrazol-1-yl)methyl]chloracetanilid

2-Methyl-6-ethyl-N-[(3-chlor-1,2,4-triazolyl)methyl]chloracetanilid

2,6-Diethyl-N-[(4-chlorpyrazol-1-yl)methyl]-chloracetanilid

Weitere bevorzugt in Frage kommende Acetanilide der Formel VIII sind in den Herstellungsbeispielen aufgeführt.

Die Acetanilide der Formel VIII und deren herbizide Wirksamkeit sowie deren herbizid wirksame Säureadditionssalze und Metallsalzkomplexe sind bereits bekannt (vgl. DE-OS Nr. 2548008 und DE-OS Nr. 2704281).

In der Formel IX steht $R^{13}$ vorzugsweise für geradkettiges oder verzweigtes Alkyl mit 1 bis 6, insbesondere 1 bis 4 Kohlenstoffatomen, Halogen, insbesondere Fluor, Chlor und Brom, Halogenalkyl mit bis zu 3 Kohlenstoff- und bis zu 5 gleichen oder verschiedenen Halogenatomen, wobei als Halogene insbesondere Fluor oder Chlor stehen, beispielhaft sei Trifluormethyl genannt; ferner vorzugsweise für Alkylthio und Alkylsulfonyl mit 1 bis 4 Kohlenstoffatomen im Alkylteil sowie für Aminosulfonyl, Cyano und Nitro. $R^{14}$ und $R^{15}$ sind gleich oder verschieden und stehen vorzugsweise für Wasserstoff, geradkettiges oder verzweigtes Alkyl mit 1 bis 4 Kohlenstoffatomen, Halogen, insbesondere Fluor, Chlor und Brom, Halogenalkyl mit bis zu 3 Kohlenstoff- und bis zu 5 gleichen oder verschiedenen Halogenatomen, wobei als Halogene insbesondere Fluor und Chlor stehen sowie vorzugsweise für gegebenenfalls einfach oder mehrfach substituiertes Phenyl, wobei als Substituenten vorzugsweise die für $R^{13}$ genannten Reste in Frage kommen. $R^{16}$ steht vorzugsweise für geradkettiges oder verzweigtes Alkyl mit 1 bis 6, insbesondere 1 bis 4 Kohlenstoffatomen sowie für gegebenenfalls einfach oder mehrfach substituiertes Phenyl, wobei als Substituenten vorzugsweise in Frage kommen: Alkyl mit 1 bis 4 Kohlenstoffatomen, Halogen, insbesondere Fluor, Chlor und Brom, Halogenalkyl mit bis zu 3 Kohlenstoffatomen und bis zu 5 gleichen oder verschiedenen Halogenatomen, insbesondere Fluor- oder Chloratome, wobei Trifluormethyl beispielhaft genannt sei, ferner Alkoxy, Alkylthio und Alkylsulfonyl mit jeweils 1 bis 4 Kohlenstoffatomen, weiterhin Aminosulfonyl, Cyano und Nitro sowie gegebenenfalls durch Chlor substituiertes Phenyl oder Phenoxy. Der Index m steht vorzugsweise für 1, 2 oder 3.

Als Beispiele für Acetanilide der Formel IX seien im einzelnen genannt:

2,6-Dimethyl-N-(benzoylmethyl)chloracetanilid

2,6-Dimethyl-N-(4-chlorbenzoylmethyl)chloracetanilid

2-Methyl-6-ethyl-N-(benzoylmethyl)chloracetanilid

Weitere bevorzugt in Frage kommende Acetanilide der Formel IX sind in den Herstellungsbeispielen aufgeführt.

Die Acetanilide der Formel IX und deren herbizide Wirksamkeit sind bereits bekannt (vgl. DE-OS Nr. 2726253).

In der Formel X steht A vorzugsweise für Sauerstoff, Schwefel oder die Gruppierung $-NR^{22}$, worin $R^{22}$ vorzugsweise für Wasserstoff, geradkettiges oder verzweigtes Alkyl mit 1 bis 4 Kohlenstoffatomen sowie für Aryl mit 6 bis 10 Kohlenstoffatomen, insbesondere Phenyl, steht, wobei jeder dieser Arylreste substituiert sein kann durch Halogen, Alkyl mit 1 bis 4 Kohlenstoffatomen, Alkoxy mit 1 oder 2 Kohlenstoffatomen, Alkylthio mit 1 oder 2 Kohlenstoffatomen, Cyano, Nitro und/oder Halogenalkyl mit bis zu 2 Kohlenstoff- und bis zu 5 gleichen oder verschiedenen Halogenatomen, wobei als Halogene insbesondere Fluor oder Chlor genannt seien. $R^{19}$ steht vorzugsweise für Wasserstoff oder Methyl. $R^{20}$ steht in der Formel X vorzugsweise für Wasserstoff, geradkettiges oder verzweigtes Alkyl mit 1 bis 4 Kohlenstoffatomen, Halogenalkyl mit bis zu 3 Kohlenstoffatomen und bis zu 5 gleichen oder verschiedenen Halogenatomen, wobei als Halogene insbesondere Fluor oder Chlor stehen, beispielhaft sei Trifluormethyl genannt, ferner vorzugsweise für Alkenyl und Alkinyl mit 2 bis 4 Kohlenstoffatomen, Cycloalkyl mit 5 bis 7 Kohlenstoffatomen sowie für Halogen, insbesondere Fluor, Chlor oder Brom. $R^{20}$ steht ferner vorzugsweise für Aryl mit 6 bis 10 Kohlenstoffatomen, insbesondere Phenyl, wobei jeder dieser Arylreste substituiert sein kann durch Halogen, Alkyl mit 1 bis 4 Kohlenstoffatomen, Alkoxy mit 1 oder 2 Kohlenstoffatomen, Alkylthio mit 1 oder 2 Kohlenstoffatomen, Cyano, Nitro und/oder Halogenalkyl mit bis zu 2 Kohlenstoffatomen und bis zu 5 gleichen oder verschiedenen Halogenatomen, wobei als Halogene insbesondere Fluor oder Chlor stehen und Trifluormethyl als Beispiel für Halogenalkyl speziell genannt sei. $R^{20}$ steht ferner vorzugsweise für Aralkyl mit 6 bis 10 Kohlenstoffatomen im Arylteil und 1 bis 4 Kohlenstoffatomen im Alkylteil, insbesondere für Benzyl, wobei jeder dieser Aralkylreste im Arylteil substituiert sein kann durch Halogen, Alkyl mit 1 bis 4 Kohlenstoffatomen, Alkoxy mit 1 oder 2 Kohlenstoffatomen, Alkylthio mit 1 oder 2 Kohlenstoffatomen, Cyano, Nitro und/oder Halogenalkyl mit bis zu 2 Kohlenstoffatomen und bis zu 5 gleichen oder verschiedenen Halogenatomen, wobei als Halogene insbesondere Fluor oder Chlor stehen und Trifluormethyl als Beispiel für Halogenalkyl speziell genannt sei. Ausserdem steht $R^{20}$ für die Gruppierungen $-OR^{23}$, $-SR^{23}$ und $-NR^{22}R^{23}$, worin $R^{22}$ für diejenigen Reste steht, die oben bereits vorzugsweise für diesen Rest genannt wurden. $R^{23}$ steht in diesen Gruppierungen für Wasserstoff, geradkettiges oder verzweigtes Alkyl mit 1 bis 4 Kohlenstoffatomen, Halogenalkyl mit 1 bis 3 Kohlenstoffatomen und bis zu 5 gleichen oder verschiedenen Halogenatomen, wobei als Halogene insbesondere Fluor oder Chlor stehen, beispielhaft sei Trifluormethyl genannt, ferner vorzugsweise für Alkenyl und Alkinyl mit 2 bis 4 Kohlenstoffatomen, Cycloalkyl mit 5 bis 7 Kohlenstoffatomen sowie für Aralkyl mit 6 bis 10 Kohlenstoffatomen im Arylteil und 1 bis 4 Kohlenstoffatomen im Alkylteil, insbesondere für Benzyl, wobei jeder dieser Aralkylreste im Arylteil substituiert sein kann durch Halogen, Alkyl mit 1 bis 4 Kohlenstoffatomen, Alkoxy mit 1 oder 2 Kohlenstoffatomen, Alkylthio mit 1 oder 2 Kohlenstoffatomen, Cyano, Nitro und/oder Halogenalkyl mit bis zu 2 Kohlenstoffatomen und bis zu 5 gleichen oder verschiedenen Halogenatomen, wobei als Halogene insbesondere Fluor oder Chlor stehen und Trifluormethyl als Beispiel für Halogenalkyl speziell genannt sei. In der Formel X steht $R^{17}$ vorzugsweise für geradkettiges

oder verzweigtes Alkyl mit 1 bis 4 Kohlenstoffatomen. $R^{18}$ steht in der Formel X vorzugsweise für geradkettiges oder verzweigtes Alkyl mit 1 bis 4 Kohlenstoffatomen oder für die Halogene Fluor, Chlor und Brom. $R^{21}$ steht in der Formel X vorzugsweise für Chlor, Brom und Jod. Der Index p steht für die Zahlen 0, 1 oder 2.

Als Beispiele für Acetanilide der Formel X seien im einzelnen genannt:

2,6-Diethyl-N-[(2-methyl-1,3,4-oxadiazol-5-yl)methyl]chloracetanilid

2,6-Dimethyl-N-[(2-methyl-1,3,4-oxadiazol-5-yl)methyl]chloracetanilid

2-Ethyl-6-methyl-N-[(2-methyl-1,3,4-oxadiazol-5-yl)methyl]chloracetanilid

2-tert.-Butyl-N-[(2-methyl-1,3,4-oxadiazol-5-yl)methyl]chloracetanilid

Weitere bevorzugt in Frage kommende Acetanilide der Formel X sind in den Herstellungsbeispielen aufgeführt.

Die Acetanilide der Formel X und deren herbizide Wirksamkeit sind bereits bekannt (vgl. EP-OS Nr. 3539).

Weitere bevorzugt in Frage kommende Acetanilide, bei denen die erfindungsgemässen Verbindungen der Formel I als Gegenmittel eingesetzt werden können, sind die Verbindungen der Formeln XI und XII. Diese Stoffe und deren herbizide Wirksamkeit sind bereits bekannt (vgl. US-PS Nr. 3442945 und DE-OS Nr. 2328340).

Die erfindungsgemässen N-Acylpiperidonketale der Formel I eignen sich insbesondere zum Schutz von wichtigen Kulturpflanzen, wie Mais, Sojabohnen, Baumwolle, Zuckerrüben, Getreide, Reis und Zuckerrohr vor Herbizidschädigungen durch Thiolcarbamate und Acetanilide.

Die erfindungsgemässen Wirkstoffkombinationen bestehend aus einem N-Acylpiperidonketal der Formel I und mindestens einem herbizid wirksamen Thiolcarbamat bzw. mindestens einem herbizid wirksamen Acetanilid zeigen eine sehr gute Wirkung gegen Unkräuter und Ungräser in zahlreichen Nutzpflanzenkulturen. Sie können daher zur selektiven Unkrautbekämpfung in zahlreichen Nutzpflanzenkulturen verwendet werden. (Unter Unkräutern im weitesten Sinne sind hierbei alle Pflanzen zu verstehen, die an Orten wachsen, wo sie unerwünscht sind.)

Die erfindungsgemässen Wirkstoffkombinationen können z.B. bei folgenden Pflanzen verwendet werden.

Dikotyle Unkräuter der Gattungen: *Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.*

Dicotyle Kulturen der Gattungen: *Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.*

Monokotyle Unkräuter der Gattungen: *Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.*

Monokotyle Kulturen der Gattungen: *Oryza, Zea, Triticum, Hordeum, Avena, Secale, Sorghum, Panicum, Saccharum, Ananas, Asparagus, Allium.*

Insbesondere eignen sich die erfindungsgemässen Wirkstoffkombinationen zur selektiven Unkrautbekämpfung in Mais, Sojabohnen, Baumwolle, Zuckerrüben, Getreide, Reis und Zuckerrohr.

Die erfindungsgemässen Gegenmittel können gegebenenfalls im Gemisch mit den herbiziden Wirkstoffen, bei denen sie eingesetzt werden, in die üblichen Formulierungen übergeführt werden, wie Lösungen, Emulsionen, Spritzpulver, Suspensionen, Pulver, Stäubemittel, Schäume, Pasten, lösliche Pulver, Granulate, Suspensions/Emulsionskonzentrate, wirkstoffimprägnierte Natur- und synthetische Stoffe, Feinstverkapselungen in polymeren Stoffen und in Hüllmassen für Saatgut.

Diese Formulierungen werden in bekannter Weise hergestellt, z.B. durch Vermischen der erfindungsgemässen Gegenmittel gegebenenfalls im Gemisch mit den herbiziden Wirkstoffen, bei denen sie eingesetzt werden, mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glykol sowie deren Ether und Ester, Ketone, wie Aceton, Methylethylketon, Methylisobutylketon oder Cyclohexanon, stark polare Lösungsmittel, wie Dimethylformamid und Dimethylsulfoxid sowie Wasser; als feste Trägerstoffe: natürliche Gesteinsmehle, wie Kaoline, Tonerden, Talkum, Kreide, Quarz, Attapulgit, Montmorillonit oder diatomeenerde und synthetische Gesteinsmehle, wie hochdisperse Kieselsäure, Aluminiumoxid und Silikate; als feste Trägerstoffe für Granulate: gebrochene und fraktionierte natürliche Gesteine wie Calcit, Marmor, Bims, Sepiolith, Dolomit sowie synthetische Granulate aus anorganischen Mehlen sowie Granulate aus organischem Material wie Sägemehle, Kokosnussschalen, Maiskolben und Tabakstengel; als Emulgier- und/oder schaumerzeugende Mittel: nicht ionogene und anionische Emulgatoren, wie Polyoxyethylen/Fettsäure/Ester, Polyoxyethylen/Fettalkohol/Ether, z.B. Alkylaryl/Polyglykol/Ether, Alkylsulfonate, Alkylsulfate, Arylsulfonate sowie Eiweisshydrolysate;

als Dispergiermittel: z.B. Ligninsulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.% an Gegenmittel bzw. an Gegenmittel und herbizidem Wirkstoff, vorzugsweise zwischen 0,5 und 90%.

Die erfindungsgemässen Gegenmittel können als solche oder in ihren Formulierungen auch in Mischung mit herbiziden Wirkstoffen eingesetzt werden, wobei Fertigformulierungen oder Tankmischung möglich ist. Auch eine Mischung mit anderen bekannten Wirkstoffen wie Fungiziden, Insektiziden, Akariziden, Nematiziden, Schutzstoffen gegen Vogelfrass, Wuchsstoffen, Pflanzennährstoffen und Bodenstruktur-Verbesserungsmitteln ist möglich.

Das erfindungsgemässe Gegenmittel bzw. Gemische aus erfindungsgemässen Gegenmitteln und herbizidem Wirkstoff können als solche, in Form ihrer Formulierungen oder der daraus durch weiteres Verdünnen bereiteten Anwendungsformen, wie gebrauchsfertige Lösungen, Suspensionen, Emulsionen, Pulver und Granulate angewandt werden. Die Anwendung geschieht in üblicher Weise, z.B. durch Giessen, Spritzen, Sprühen, Stäuben oder Streuen, Trockenbeizen, Feuchtbeizen, Nassbeizen, Schlämmbeizen oder Inkrustieren.

Die erfindungsgemässen Gegenmittel können nach den für derartige Antidote üblichen Methoden ausgebracht werden. So können die erfindungsgemässen Gegenmittel entweder vor oder nach dem Herbizid ausgebracht oder zusammen mit dem Herbizid appliziert werden. Ferner können Kulturpflanzen durch Saatgutbehandlung mit den Gegenmitteln vor der Saat (Beizung) vor Schäden geschützt werden, wenn das Herbizid vor oder nach der Saat angewendet wird. Eine weitere Einsatzmöglichkeit besteht darin, dass man die Gegenmittel bei der Aussaat in die Saatfurche ausbringt. Wenn es sich bei den Pflanzen um Stecklinge handelt, können diese vor der Auspflanzung mit den Gegenmitteln behandelt werden.

Beim Einsatz der erfindungsgemässen Gegenmittel kommen die ortsüblichen Aufwandmengen an den jeweiligen Herbiziden zur Anwendung. Die Aufwandmengen an herbizidem Wirkstoff schwanken zwischen 0,5 und 5 kg/ha. Die Aufwandmenge an Gegenmittel ist unabhängig vom Herbizid und der Aufwandmenge des herbiziden Wirkstoffes. Im allgemeinen liegen die Aufwandmengen an erfindungsgemässen Gegenmitteln bei Flächenbehandlung zwischen 0,1 und 5 kg/ha, vorzugsweise zwischen 0,2 und 4 kg/ha. Bei der Saatgutbehandlung liegen die Aufwandmengen an erfindungsgemässen Gegenmitteln im allgemeinen zwischen 10 und 300 g/kg Saatgut, vorzugsweise zwischen 25 und 200 g/kg Saatgut.

In den erfindungsgemässen Wirkstoffkombinationen können die Gewichtsverhältnisse von Gegenmitteln zu herbiziden Wirkstoffen in relativ grossen Bereichen schwanken. Im allgemeinen entfallen auf 1 Gew.-Teil an herbizidem Wirkstoff 0,04 bis 1,0 Gew.-Teil, vorzugsweise 0,1 bis 0,5 Gew.-Teil an Gegenmittel der Formel I.

Die gute Wirksamkeit der erfindungsgemässen Gegenmittel geht aus dem nachfolgenden Beispiel hervor.

In diesem Beispiel werden die nachstehend angegebenen Verbindungen als Vergleichskomponenten eingesetzt:

A = 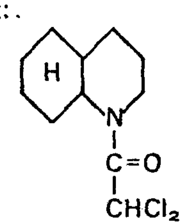

(N-Dichloracetyl-cis,trans-decahydrochinolin)

B = 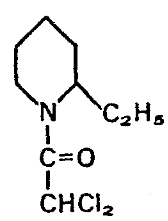

(N-Dichloracetyl-2-ethylpiperidin)

Ferner wird in diesem Beispiel als herbizider Wirkstoff das nachstehend angegebene Acetanilid eingesetzt.

C = 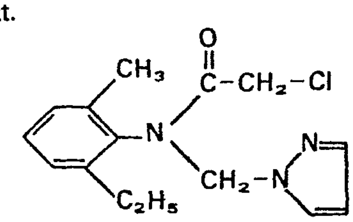

[2-Methyl-6-ethyl-N-(pyrazol-1-yl-methyl)-chloracetanilid]

*Beispiel A*

*Pre-emergence-Test*

Lösungsmittel: 5 Gew.-Teile Aceton

Emulgator: 1 Gew.-Teil Alkylarylpolyglykolether

Zur Herstellung einer zweckmässigen Wirkstoffzubereitung vermischt man 1 Gew.-Teil herbiziden Wirkstoff bzw. Gegenmittel bzw. eines Gemisches aus Gegenmittel und herbizidem Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalem Boden ausgesät und nach 24 h mit der Herbizidzubereitung bzw. mit der Gegenmittelzubereitung bzw. mit der Zubereitung aus Gegenmittel und herbizidem Wirkstoff begossen. Dabei hält man die Wassermenge pro Flächeneinheit zweckmässigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in %-Schädigung im Vergleich zur Entwicklung der unbehandelten Kontrolle. Es bedeuten:

0% = keine Wirkung (wie unbehandelte Kontrolle)
100% = totale Vernichtung

Eine Auswertung der Testergebnisse zeigt, dass die erfindungsgemässe Verbindung 1 besser zum Schutz von Kulturpflanzen vor Schäden durch das 2-Methyl-6-ethyl-N-(pyrazol-1-yl-methyl)-chloracetanilid geeignet ist als die Vergleichskomponenten A und B.

*Herstellungsbeispiele*

*Beispiel 1*

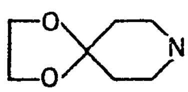

Zu einer Mischung aus 12 g (0,084 mol) Piperidon-4-ethylenketal und 12,8 g (0,84 mol) 1,8-Diazabicyclo-[5.4.0]-undec-7-en in 100 ml Toluol werden 12,4 g (0,084 mol) Dichloracetylchlorid unter Rühren so zugetropft, dass die Temperatur des Reaktionsgemisches 40° C nicht übersteigt. Danach wird 2 h bei 20° C nachgerührt. Anschliessend arbeitet man auf, indem man das Reaktionsgemisch filtriert, das Filtrat unter vermindertem Druck einengt und den verbleibenden Rückstand aus einem Gemisch von Toluol und Petrolether umkristallisiert. Man erhält auf diese Weise 10,5 g (49% der Theorie) an N-Dichloracetylpiperidon-4-ethylenketal in Form farbloser Kristalle vom Schmelzpunkt 102° C.

*Beispiel 2*

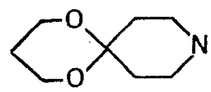

Eine Mischung aus 19 g (0,09 mol) N-Dichloracetylpiperidon-4, 7,5 g (0,099 mol) Propandiol-1,3 und 22,4 g (0,21 mol) Trimethylchlorsilan wird 12 h unter Rühren am Rückfluss gekocht. Danach wird das Reaktionsgemisch unter vermindertem Druck eingeengt und anschliessend unter stark vermindertem Druck kurz andestilliert. Das erhaltene braune Öl, das langsam durchkristallisiert, wird aus 200 ml Toluol umkristallisiert. Man erhält 16 g (66,6% der Theorie) an N-Dichloracetylpiperidon-4-propylenketal vom Schmelzpunkt 115° C.

*Analyse:*

| | | | | |
|---|---|---|---|---|
| berechnet: | C 44,7 | H 5,5 | Cl 26,4 | N 5,2% |
| gefunden: | C 44,5 | H 5,6 | Cl 26,0 | N 5,7% |

Nach den in den Beispielen 1 und 2 beschriebenen Methoden werden auch die in der nachfolgenden Tabelle 1 aufgeführten N-Acylpiperidonketale der Formel I hergestellt.

*Tabelle 1*

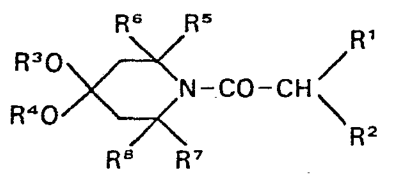

| Beispiel Nr. | $R^1$ | $R^2$ | $R^3$ | $R^4$ | $R^5$ | $R^6$ | $R^7$ | $R^8$ | Brechungsindex bzw. Schmelzpunkt (° C) |
|---|---|---|---|---|---|---|---|---|---|
| 3 | Cl | Cl | $CH_3$ | $CH_3$ | H | H | H | H | 76 |
| 4 | Cl | Cl | $C_2H_5$ | $C_2H_5$ | H | H | H | H | 64 |
| 5 | $CH_3$ | Cl | $-CH_2-$ | $-CH_2-$ | H | H | H | H | $n_D^{23}$ = 1,5059 |
| 6 | $CH_3$ | Cl | $-CH_2-CH_2-$ | $-CH_2-$ | H | H | H | H | 70 |
| 7 | $CH_3$ | Cl | $C_2H_5$ | $C_2H_5$ | H | H | H | H | $n_D^{23}$ = 1,5091 |

*Beispiel (VIII-1)*

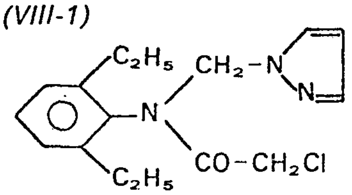

Zu 274,2 g (1 mol) 2,6-Diethyl-N-chlormethylchloracetanilid in 250 ml wasserfreiem Essigester gibt man unter Rühren eine Mischung aus 68 g (1 mol) Pyrazol und 106 g (1,05 mol) Triethylamin in 150 ml wasserfreien Essigester, wobei die Temperatur auf 30° C ansteigt. Man rührt 1 h bei Raumtemperatur nach. Für die Aufarbeitung ergeben sich zwei Möglichkeiten:

1) Das Reaktionsgemisch wird filtriert, das Filtrat mit Wasser neutral gewaschen, über Natriumsulfat getrocknet und im Vakuum eingedampft.

Nach einer fraktionierten Kristallisation mit Ligroin erhält man 171,2 g (56% der Theorie) 2,6-Diethyl-N-(pyrazol-1-yl-methyl)chloracetanilid vom Schmelzpunkt 67° C in Form farbloser Kristalle.

2) Das Reaktionsgemisch wird auf 0° C abgekühlt, filtriert und der Filterrückstand mit 10 ml kaltem Essigester nachgewaschen. In das Filtrat werden bei 0 bis −10° C 50 g (1,4 mol) trockener Chlorwasserstoff eingeleitet. Man saugt anschliessend die ausgefallenen Hydrochloridsalze ab, wäscht mit 50 ml kaltem Essigester nach und verteilt den festen Rückstand zwischen 0,5 l Essigester und 0,5 l wässriger Natriumhydroxidlösung mit einem pH-Wert von 12. Die organische Phase wird abgetrennt, zweimal mit je 0,5 l Natriumchloridlösung gewaschen, über Natriumsulfat getrocknet und im Vakuum eingedampft. Der farblose ölige Rückstand wird mit 60 ml Benzin versetzt, wobei er kristallisiert. Man erhält 220,2 g (72% der Theorie) 2,6-Diethyl-N-(pyrazol-1-yl-methyl)chloracetanilid vom Schmelzpunkt 67° C in Form farbloser Kristalle.

In analoger Weise werden die in der nachfolgenden Tabelle aufgeführten Verbindungen hergestellt:

*Tabelle 2*

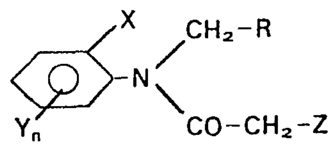

(VIII)

| Beispiel Nr. | X | Yn | Z | R | Schmelzpunkt (° C) |
|---|---|---|---|---|---|
| VIII-2 | $C_2H_5$ | 6-$C_2H_5$ | Cl | 1,2,4-Triazol-1-yl | 112 |
| VIII-3 | i-$C_3H_7$ | 6-i-$C_3H_7$ | Cl | Pyrazol-1-yl | 134 |
| VIII-4 | $CH_3$ | 6-$C_2H_5$ | Cl | 1,2,4-Triazol-1-yl | 92 |
| VIII-5 | $CH_3$ | 6-$C_2H_5$ | Cl | Pyrazol-1-yl | 57 |
| VIII-6 | $C_2H_5$ | 4,6-$(CH_3)_2$ | Cl | Pyrazol-1-yl | 32 |
| VIII-7 | $CH_3$ | 4,6-$(CH_3)_2$ | Cl | Pyrazol-1-yl | 92 |
| VIII-8 | $C_2H_5$ | 4-$CH_3$, 6-$C_2H_5$ | Cl | Pyrazol-1-yl | 78 |
| VIII-9 | i-$C_3H_7$ | 6-i-$C_3H_7$ | Cl | 1,3,4-Triazol-1-yl | 196 |
| VIII-10 | i-$C_3H_7$ | 6-i-$C_3H_7$ | Cl | 1,2,4-Triazol-1-yl | 138 |
| VIII-11 | $C_2H_5$ | 6-$C_2H_5$ | Cl | Pyrrol-1-yl | Öl |
| VIII-12 | i-$C_3H_7$ | — | Cl | 1,2,4-Triazol-1-yl | 118 |
| VIII-13 | $CH_3$ | 6-$C_2H_5$ | Cl | 1,2,3,4-Tetrazol-1-yl | Öl |
| VIII-14 | i-$C_3H_7$ | — | Cl | Pyrazol-1-yl | Öl |
| VIII-15 | $C_2H_5$ | — | Cl | 1,2,4-Triazol-1-yl | 81 |
| VIII-16 | $CH_3$ | 6-$CH_3$ | Cl | Pyrazol-1-yl | 82 |
| VIII-17 | $CH_3$ | 6-$CH_3$ | Cl | 1,2,4-Triazol-1-yl | 110 |
| VIII-18 | $CH_3$ | 5-$CH_3$ | Cl | 1,2,4-Triazol-1-yl | Öl |
| VIII-19 | $CH_3$ | — | Cl | Pyrazol-1-yl | 56 |
| VIII-20 | $CH_3$ | — | Cl | 1,2,4-Triazol-1-yl | 88 |
| VIII-21 | $CH_3$ | 5-$CH_3$ | Cl | Pyrazol-1-yl | Öl |
| VIII-22 | $CH_3$ | 3-$CH_3$ | Cl | 1,2,4-Triazol-1-yl | 114 |
| VIII-23 | $CH_3$ | 3-$CH_3$ | Cl | Pyrazol-1-yl | 102 |
| VIII-24 | $C_2H_5$ | 6-$CH_3$ | Cl | Pyrazol-1-yl (×HCl) | 87 |
| VIII-25 | $C_2H_5$ | 6-$C_2H_5$ | Cl | Pyrazol-1-yl (×HCl) | 67 |
| VIII-26 | $C_2H_5$ | 6-$C_2H_5$ | Cl | 3,5-Dimethylpyrazol-1-yl | 111 |
| VIII-27 | $C_2H_5$ | 6-$C_2H_5$ | Cl | Brommethylpyrazolyl | 145 |
| VIII-28 | $C_2H_5$ | 6-$C_2H_5$ | Cl | 3-Chlor-1,2,4-triazol-1-yl | 110 |
| VIII-29 | $CH_3$ | 6-$C_2H_5$ | Cl | 3,5-Dimethylpyrazol-1-yl | 90 |
| VIII-30 | $C_2H_5$ | 6-$C_2H_5$ | Cl | 3-Methylpyrazol-1-yl | 89 |
| VIII-31 | $C_2H_5$ | 6-$CH_3$ | Cl | 3-Methylpyrazol-1-yl | 113 |
| VIII-32 | $C(CH_3)_3$ | — | Cl | Pyrazol-1-yl | Öl |
| VIII-33 | $C(CH_3)_3$ | — | Cl | 1,2,4-Triazol-1-yl | 118 |
| VIII-34 | $C_2H_5$ | 6-$CH_3$ | Cl | Brommethylpyrazolyl | 80 |
| VIII-35 | $CH_3$ | 6-$C_2H_5$ | Cl | 4-Chlorpyrazol-1-yl | 91 |
| VIII-36 | $CH_3$ | 6-$C_2H_5$ | Cl | 3-Chlor-1,2,4-triazol-1-yl | 121 |
| VIII-37 | $C_2H_5$ | 6-$CH_3$ | Cl | 2,4,5-Trichlorimidazol-1-yl | 158 |
| VIII-38 | $C_2H_5$ | 6-$C_2H_5$ | Cl | 4-Chlorpyrazol-1-yl | 110 |
| VIII-39 | $C_2H_5$ | 6-$C_2H_5$ | Cl | 1,2,3,4-Tetrazol-1-yl | 110 |
| VIII-40 | $C_2H_5$ | 6-$C_2H_5$ | Br | Pyrazol-1-yl | 68 |
| VIII-41 | $CH_3$ | 6-$C_2H_5$ | Br | Pyrazol-1-yl | 67 |

*Tabelle 2* (Fortsetzung)

| Beispiel Nr. | X | Yn | Z | R | Schmelzpunkt (°C) |
|---|---|---|---|---|---|
| VIII-42 | $C_2H_5$ | 6-$C_2H_5$ | Cl | Imidazol-1-yl | Öl |
| VIII-43 | $C_2H_5$ | 6-$C_2H_5$ | Br | 1,2,4-Triazol-1-yl | 90 |
| VIII-44 | $CH_3$ | 6-$C_2H_5$ | Br | 1,2,4-Triazol-1-yl | 78 |

*Beispiel (IX-1)*

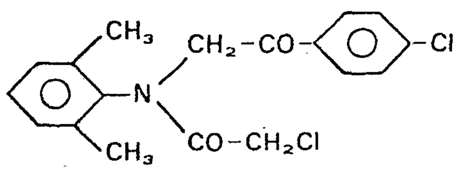

In eine Lösung von 18,5 g (0,068 mol) 2,6-Dimethyl-N-(4-chlorbenzylmethyl)anilin in 150 ml Benzol werden 16 ml (0,2 mol) Chloracetylchlorid getropft. Danach lässt man 15 h unter Rückfluss rühren und engt durch Abdestillieren des Lösungsmitels und des überschüssigen Chloracetylchlorids im Vakuum ein. Der Rückstand wird mit einem Gemisch Ether/Petrolether (1:3) verrieben, der entstehende kristalline Rückstand abgesaugt und getrocknet. Man erhält 17,7 g (75,5% der Theorie) 2,6-Dimethyl-N-(4-chlorbenzoylmethyl)chloracetanilid vom Schmelzpunkt 128°C.

*Beispiel (IX-2)*

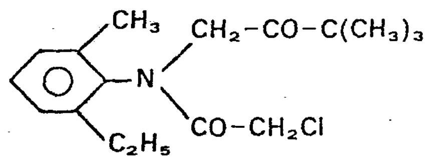

23,3 g (0,1 mol) 2-Ethyl-6-methyl-N-pivaloylmethylanilin werden in 100 ml Benzol gelöst und mit 24 ml (0,3 mol) Chloracetylchlorid versetzt. Danach lässt man 15 h unter Rückfluss rühren und engt durch Abdestillieren des Lösungsmittels und des überschüssigen Chloracetylchlorids im Vakuum ein. Der ölige Rückstand wird mit Petrolether verrührt, dekantiert, mit Aktivkohle verrührt, filtriert und im Vakuum eingeengt. Der Rückstand wird mit n-Hexan verrührt, der resultierende Feststoff abgesaugt und getrocknet. Man erhält 13,7 g (45% der Theorie) 2-Ethyl-6-methyl-N-pivaloylmethylchloracetanilid vom Schmelzpunkt 86°C.

Nach der in den Beispielen (IX-1) und (IX-2) beschriebenen Methode werden auch die in der nachfolgenden Tabelle 3 aufgeführten Verbindungen dargestellt:

Tabelle 3

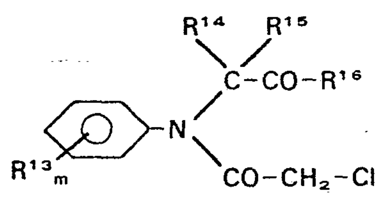

(IX)

| Bsp.-Nr. | $R^{13}_m$ | $R^{14}$ | $R^{15}$ | $R^{16}$ | Schmelzpunkt (°C) bzw. Brechungsindex |
|---|---|---|---|---|---|
| IX-3 | 2-$CH_3$ | H | H | 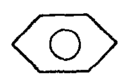 | 138 |
| IX-4 | 2-$CH_3$ | H | H | 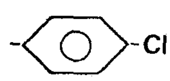 | 140 |
| IX-5 | 2,6-$(C_2H_5)_2$ | H | H | 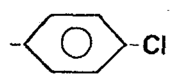 | 134 |
| IX-6 | 2,6-$(C_2H_5)_2$ | H | H | 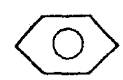 | 116 |
| IX-7 | 2-Cl | H | H | 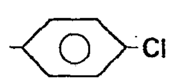 | 124 |
| IX-8 | 2,6-$(CH_3)_2$ | H | H | 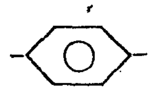 | 100 |

Tabelle 3 (Fortsetzung)

| Bsp.-Nr. | $R^{13}{}_m$ | $R^{14}$ | $R^{15}$ | $R^{16}$ | Schmelzpunkt (°C) bzw. Brechungsindex |
|---|---|---|---|---|---|
| IX-9 | 4-Cl | H | H | 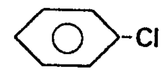 | 114 |
| IX-10 | 2,6-$(CH_3)_2$ | $CH_3$ | H | $CH_3$ | 104 |
| IX-11 | 2,6-$(i\text{-}C_3H_7)_2$ | H | H | 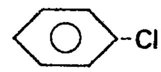 | 200 |
| IX-12 | 2,6-$(X_2H_5)_2$, 4-$CH_3$ | H | H | | 112 |
| IX-13 | 2,6-$(i\text{-}C_3H_7)_2$ | H | H | 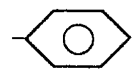 | 140 |
| IX-14 | 2,6-$(CH_3)_2$ | H | H | 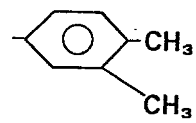 | 90 |
| IX-15 | 2-$C_2H_5$, 6-$CH_3$ | H | H | 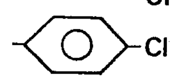 | 70 |
| IX-16 | 2,6-$(CH_2)_2$ | H | H | 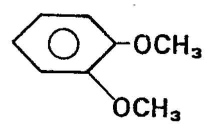 | 114 |
| IX-17 | 2-$C_2H_5$, 4,6-$(CH_3)_2$ | H | H | 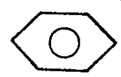 | $n_D^{20}$ = 1,5680 |
| IX-18 | 2,6-$(CH_3)_2$ | H | H | 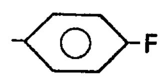 | 104 |
| IX-19 | 2,4,6-$(CH_3)_3$ | H | H | 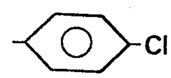 | 134 |
| IX-20 | 2,4,6-$(CH_3)_3$ | H | H | 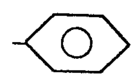 | $n_D^{20}$ = 1,5610 |
| IX-21 | 2,6-$(CH_3)_2$ | H | 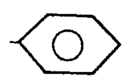 | 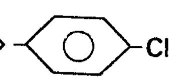 | 149 |
| IX-22 | 2,6-$(CH_3)_2$ | H | $CH_3$ | 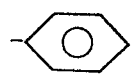 | 84 |

In analoger Weise können die in der nachfolgenden Tabelle 4 aufgeführten Verbindungen erhalten werden.

Tabelle 4

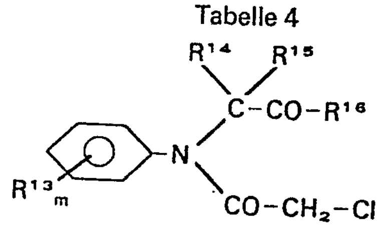

(IX)

| Bsp.-Nr. | $R^{13}{}_m$ | $R^{14}$ | $R^{15}$ | $R^{16}$ |
|---|---|---|---|---|
| IX-23 | 3,5-$(CF_3)_2$ | H | H | 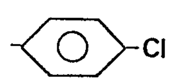 |

Tabelle 4 (Fortsetzung)

| Bsp.-Nr. | $R^{13}_m$ | $R^{14}$ | $R^{15}$ | $R^{16}$ |
|---|---|---|---|---|
| IX-24 | 2,6-$(CH_3)_2$ | H | H | 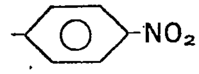 |
| IX-25 | 2,6-$(CH_3)_2$ | H | H | 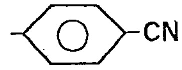 |
| IX-26 | 2,6-$(CH_3)_2$ | H | H | 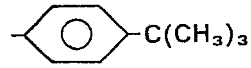 |
| IX-27 | 2,6-$(CH_3)_2$, 4-$SO_2NH_2$ | H | H | 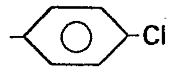 |
| IX-28 | 2-Cl, 6-$CH_3$ | H | H | 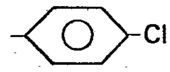 |
| IX-29 | 2-$C_2H_5$, 6-$CH_3$ | $CH_3$ | $CH_3$ | 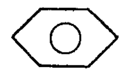 |
| IX-30 | 2-$C_2H_5$, 6-$CH_3$ | $CH_3$ | $CH_3$ | 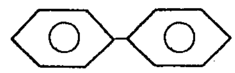 |
| IX-31 | 2-$C_2H_5$, 6-$CH_3$ | $CH_3$ | $CH_3$ | 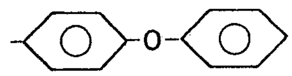 |
| IX-32 | 2-$C_2H_5$, 6-$CH_3$ | $CH_3$ | $CH_3$ | 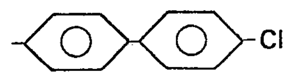 |
| IX-33 | 2-$C_2H_5$, 6-$CH_3$ | $CH_3$ | $CH_3$ | 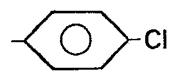 |
| IX-34 | 2-$C_2H_5$, 6-$CH_3$ | H | $CH_3$ | 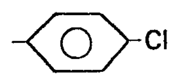 |
| IX-35 | 2-$C_2H_5$, 6-$CH_3$ | H | $CH_3$ | 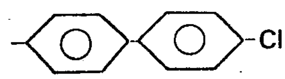 |
| IX-36 | 2,6-$(CH_3)_2$ | H | 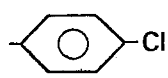 | 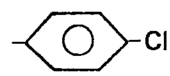 |
| IX-37 | 2,6-$(CH_3)_2$ | H | 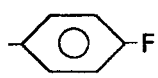 | 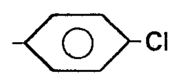 |
| IX-38 | 2,6-$(CH_3)_2$ | H | 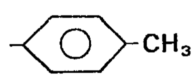 | 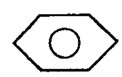 |
| IX-39 | 2,6-$(CH_3)_2$ | H | 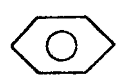 | 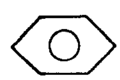 |
| IX-40 | 2,6-$(CH_3)_2$ | H | 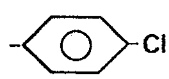 | 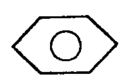 |
| IX-41 | 2,6-$(CH_3)_2$ | H | $CH_3$ | 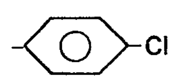 |

*Beispiel (X-1)*

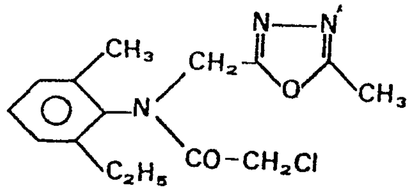

16,3 g (0,07 mol) 2-Ethyl-6-methyl-N-[(2-methyl-1,3,4-oxadiazol-6-yl)methyl]anilin und 6 g (0,076 mol) wasserfreies Pyridin werden in 100 ml absolutem Tetrahydrofuran unter Rühren zum Sieden erhitzt und tropfenweise mit einer Lösung von 8 g (0,07 mol) Chloracetylchlorid in 20 ml Tetrahydrofuran versetzt. Nach beendetem Zutropfen lässt man 10 min nachrühren, engt durch Abdestillieren des Lösungsmittels ein und verrührt den Rückstand mit 150 ml Wasser. Das auskristallisierende Reaktionsprodukt wird abgesaugt, mit Wasser gewaschen und getrocknet. Man erhält 18,7 g (87% der Theorie) beige-farbene Kristalle von 2-Ethyl-6-methyl-N-[(2-methyl-1,3,4-oxadiazol-5-yl)methyl]chloracetanilid vom Schmelzpunkt 67 bis 70° C.

*Beispiel (X-2)*

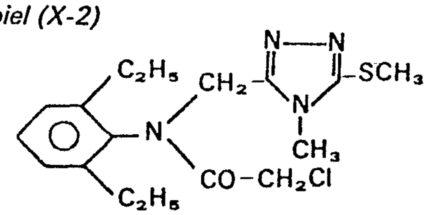

5 g (0,017 mol) 2,6-Diethyl-N-[(1-methyl-2-methylthio-1,3,4-triazol-5-yl)methyl]anilin und 1,6 g (0,02 mol) Pyridin werden in 100 ml absolutem Tetrahydrofuran gerührt und bei Raumtemperatur tropfenweise mit 2,3 g (0,02 mol) Chloracetylchlorid versetzt, wobei die Temperatur auf ca. 30° C ansteigt. Man lässt 2 h rühren, engt teilweise durch Abdestillieren des Lösungsmittels ein und versetzt mit Wasser. Das auskristallisierende Produkt wird abgesaugt, getrocknet und aus Diisopropylether/Essigester umkristallisiert. Man erhält 5 g (80% der Theorie) 2,6-Diethyl-N-[(1-methyl-2-methylthio-1,3,4-triazol-5-yl)methyl]-chloracetanilid vom Schmelzpunkt 121 bis 123° C.

In entsprechender Weise werden diejenigen Verbindungen erhalten, die in der Tabelle 5 formelmässig aufgeführt sind.

*Tabelle 5*

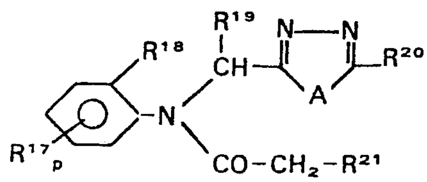
(X)

| Beispiel Nr. | $R^{19}$ | $R^{20}$ | $R^{18}$ | $R^{17}p$ | A | $R^{21}$ | Schmelzpunkt (° C) |
|---|---|---|---|---|---|---|---|
| X-3 | H | $CH_3$ | $C_2H_5$ | 6-$C_2H_5$ | O | Cl | 79-82 |
| X-4 | H | $CH_3$ | $CH_3$ | 6-$CH_3$ | O | Cl | 91-93 |
| X-5 | H | $CH_3$ | $C(CH_3)_3$ | — | O | Cl | 102-04 |
| X-6 | H | $-S-CH_2-CH=CH_2$ | $C_2H_5$ | 6-$C_2H_5$ | 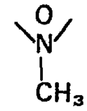 | Cl | 67-70 |
| X-7 | H | 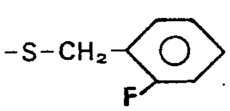 | $CH_3$ | 6-$C_3H_5$ | 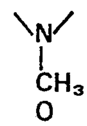 | Cl | 115-20 |
| X-8 | H | $C_2H_5$ | $CH_3$ | 6-$C_2H_5$ | O | Cl | 57-59 |
| X-9 | H | $C_2H_5$ | $C_2H_5$ | 6-$C_2H_5$ | O | Cl | 43-47 |
| X-10 | H | i-$C_3H_7$ | $CH_3$ | 6-$C_2H_5$ | O | Cl | zähflüssiges Öl |
| X-11 | H | $CH_3$ | $CH_3$ | 3-$CH_3$ | 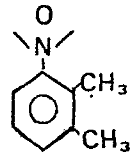 | Cl | glasartig erstarrt |
| X-12 | H | $CH_3$ | $C_2H_5$ | 6-$C_2H_5$ | | Br | 80 |
| X-13 | H | $CH_3$ | $CH_3$ | 6-$C_2H_5$ | | Br | 92-94 |
| X-14 | H | $CH_3$ | i-$C_3H_7$ | 6-i-$C_3H_7$ | | Cl | 135-37 |

**Patentansprüche**

1. N-Acylpiperidonketale der Formel

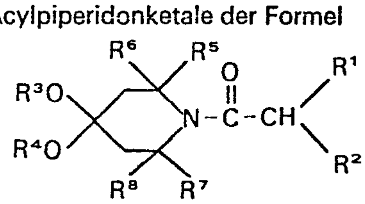
(I)

in welcher
$R^1$ für Alkyl oder Halogen steht,
$R^2$ für Halogen steht,
$R^3$ für Alkyl steht,
$R^4$ für Alkyl steht, oder
$R^3$ und $R^4$ gemeinsam für eine gegebenenfalls verzweigte Alkylenkette stehen, und
$R^5$, $R^6$, $R^7$ und $R^8$ unabhängig voneinander für Wasserstoff oder Alkyl stehen.

2. Verfahren zur Herstellung von N-Acylpiperidonketalen der Formel (I), dadurch gekennzeichnet, dass man

a) Piperidonketale der Formel

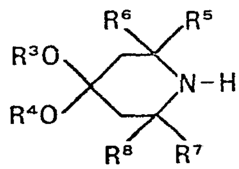

(II)

in welcher

$R^3$, $R^4$, $R^5$, $R^6$, $R^7$ und $R^8$ die oben angegebene Bedeutung haben,

mit Alkanoylchloriden der Formel

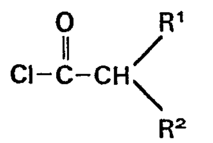

(III)

in welcher

$R^1$ und $R^2$ die oben angegebene Bedeutung haben,

gegebenenfalls in Gegenwart eines Verdünnungsmittels sowie gegebenenfalls in Gegenwart eines Säurebindemittels umsetzt,

oder

b) N-Acylpiperidone der Formel

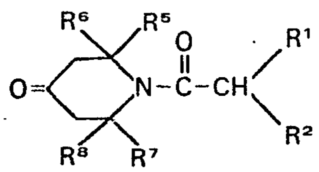

(IV)

in welcher

$R^1$, $R^2$, $R^5$, $R^6$, $R^7$ und $R^8$ die oben angegebene Bedeutung haben,

mit Alkoholen der Formel

$$R^9-OH \qquad (V)$$

in welcher

$R^9$ für Alkyl oder ω-Hydroxyalkyl steht,

in Gegenwart eines Katalysators oder eines Wasserbindemittels sowie gegebenenfalls in Gegenwart eines Verdünnungsmittels umsetzt.

3. Mittel zum Schutz von Kulturpflanzen vor Schädigungen durch herbizid wirksame Thiolcarbamate bzw. durch herbizid wirksame Acetanilide, gekennzeichnet durch einen Gehalt an mindestens einem N-Acylpiperidonketal der Formel (I) gemäss Anspruch 1.

4. Verfahren zum Schutz von Kulturpflanzen vor Schädigungen durch herbizid wirksame Thiolcarbamate bzw. herbizid wirksame Acetanilide, dadurch gekennzeichnet, dass man N-Acylpiperidonketale der Formel (I) gemäss Anspruch 1 auf die Kulturpflanzen und/oder deren Lebensraum einwirken lässt.

5. Verwendung von N-Acylpiperidonketalen der Formel (I) gemäss Anspruch 1 zum Schutz von Kulturpflanzen vor Schädigungen durch herbizid wirksame Thiolcarbamate bzw. durch herbizid wirksame Acetanilide.

6. Verfahren zur Herstellung von Mitteln zum Schutz von Kulturpflanzen vor Schädigungen durch herbizid wirksame Thiolcarbamate bzw. herbizid wirksame Acetanilide, dadurch gekennzeichnet, dass man N-Acylpiperidonketale der Formel (I) gemäss Anspruch 1 mit Streckmitteln und/oder oberflächenaktiven Mitteln vermischt.

7. Mittel zur selektiven Unkrautbekämpfung in Nutzpflanzenkulturen, gekennzeichnet durch einen Gehalt an einer Wirkstoffkombination, bestehend aus mindestens einem N-Acylpiperidonketal der Formel (I) gemäss Anspruch 1 und mindestens einem herbizid wirksamen Thiolcarbamat bzw. mindestens einem herbizid wirksamen Acetanilid.

8. Verwendung von Wirkstoffkombinationen gemäss Anspruch 5 zur selektiven Unkrautbekämpfung in Nutzpflanzenkulturen.

9. N-Acylpiperidonketal der Formel

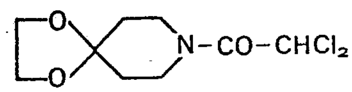

10. N-Acylpiperidonketal der Formel

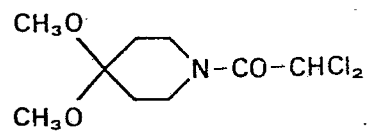

## Claims

1. N-Acyl-piperidone ketals of the formula:

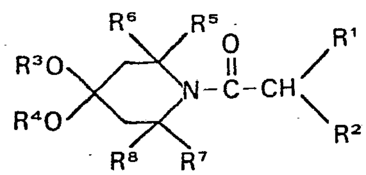

(I)

in which:

$R^1$ represents alkyl or halogen,

$R^2$ represents halogen,

$R^3$ represents alkyl,

$R^4$ represents alkyl, or

$R^3$ and $R^4$ together represent an optionally branched alkylene chain, and

$R^5$, $R^6$, $R^7$ and $R^8$ independently of one another represent hydrogen or alkyl.

2. Process for the preparation of N-acyl-piperidone ketals of the formula (I), characterized in that a) piperidone ketals of the formula:

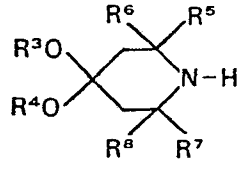

(II)

in which:

$R^3$, $R^4$, $R^5$, $R^6$, $R^7$ and $R^8$ have the meaning indicated above,

are reacted with alkanoyl chlorides of the formula

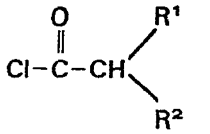 (III)

in which:

$R^1$ and $R^2$ have the meaning indicated above, if appropriate in the presence of a diluent and if appropriate in the presence of an acid-binding agent, or

b) N-acyl-piperidones of the formula:

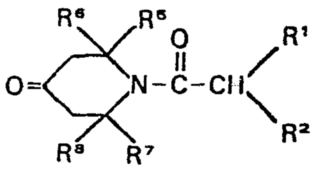 (IV)

in which:

$R^1$, $R^2$, $R^5$, $R^6$, $R^7$ and $R^8$ have the meaning indicated above,
are reacted with alcohols of the formula:

$$R^9{-}OH \quad (V)$$

in which:

$R^9$ represents alkyl or ω-hydroxyalkyl,
in the presence of a catalyst or of a water-binding agent, and if appropriate in the presence of a diluent.

3. Agents for protecting crop plants from damage by herbicidally active thiolcarbamates or by herbicidally active acetanilides, characterized in that they contain at least one N-acyl-piperidone ketal of the formula (I) according to claim 1.

4. Process for protecting crop plants from damage by herbicidally active thiolcarbamates or herbicidally active acetanilides, characterized in that N-acyl-piperidone ketals of the formula (I) according to claim 1 are allowed to act on the crop plants and/or their environment.

5. Use of N-acyl-piperidone ketals of the formula (I) according to claim 1 for protecting crop plants from damage by herbicidally active thiolcarbamates or by herbicidally active acetanilides.

6. Process for the preparation of agents for protecting crop plants from damage by herbicidally active thiolcarbamates or herbicidally active acetanilides, characterized in that N-acyl-piperidone ketals of the formula (I) according to claim 1 are mixed with extenders and/or surface-active agents.

7. Agents for selectively combating weeds in crops of useful plants, characterized in that they contain an active compound combination consisting of at least one N-acyl-piperidone ketal of the formula (I) according to claim 1 and at least one herbicidally active thiolcarbamate or at least one herbicidally active acetanilide.

8. Use of active compound combinations according to claim 5 for selectively combating weeds in crops of useful plants.

9. N-Acyl-piperidone ketal of the formula:

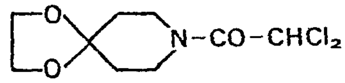

10. N-Acyl-piperidone ketal of the formula:

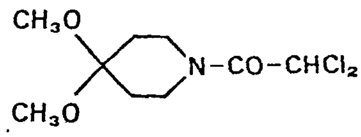

**Revendications**

1. Acétals de N-acylpipéridones de formule:

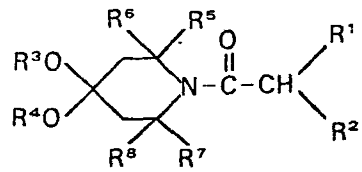 (I)

dans laquelle:

$R^1$ représente un groupe alkyle ou un halogène,
$R^2$ représente un halogène,
$R^3$ représente un groupe alkyle,
$R^4$ représente un groupe alkyle, ou
$R^3$ et $R^4$ forment ensemble une chaîne alkylène éventuellement ramifiée, et
$R^5$, $R^6$, $R^7$ et $R^8$ représentent, indépendamment les uns des autres, l'hydrogène ou des groupes alkyle.

2. Procédé de préparation des acétals de N-acylpipéridones de formule I, caractérisé en ce que:

a) on fait réagir des acétals de pipéridones de formule:

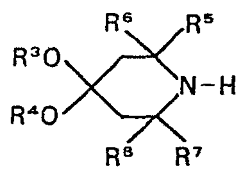 (II)

dans laquelle $R^3$, $R^4$, $R^5$, $R^6$, $R^7$ et $R^8$ ont les significations indiquées ci-dessus,
avec des chlorures d'alcanoyle de formule:

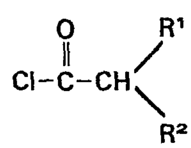 (III)

dans laquelle:

$R^1$ et $R^2$ ont les significations indiquées ci-dessus, éventuellement en présence d'un diluant et éventuellement en présence d'un agent fixant les acides, ou

b) on fait réagir des N-acylpipéridones de formule:

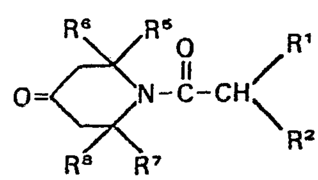 (IV)

dans laquelle:

$R^1$, $R^2$, $R^5$, $R^6$, $R^7$ et $R^8$ ont les significations indiquées ci-dessus,

avec des alcools de formule:

$$R^9{-}OH \quad (V)$$

dans laquelle:

$R^9$ représente un groupe alkyle ou ω-hydroxy-alkyle

en présence d'un catalyseur ou d'un agent déshydratant et éventuellement en présence d'un diluant.

3. Produit pour la protection des végétaux cultivés contre les dégâts provoqués par des thiolcarbamates à activité herbicide ou par des acétanilides à activité herbicide, caractérisé en ce qu'il contient au moins un acétal de N-acylpipéridone de formule I selon la revendication 1.

4. Procédé pour protéger les végétaux cultivés contre les dégâts provoqués par des thiolcarbamates à activité herbicide ou des acétanilides à activité herbicide, caractérisé en ce que l'on fait agir sur les végétaux cultivés et/ou leur habitat des acétals de N-acylpipéridones de formule I selon la revendication 1.

5. Utilisation des acétals de N-acylpipéridones de formule I selon la revendication 1 pour la protection des végétaux cultivés contre les dégâts provoqués par des thiolcarbamates à activité herbicide ou par des acétanilides à activité herbicide.

6. Procédé de préparation de produits pour la protection des végétaux cultivés contre les dégâts provoqués par des thiolcarbamates à activité herbicide ou des acétanilides à activité herbicide, caractérisé en ce que l'on mélange des acétals de N-acylpipéridones de formule I selon la revendication 1 avec des diluants et/ou des agents tensio-actifs.

7. Produit pour lutter sélectivement contre les mauvaises herbes dans les cultures de végétaux utiles, caractérisé en ce qu'il contient une combinaison de substances actives consistant en au moins un acétal de N-acylpipéridone de formule I selon la revendication 1 et au moins un thiolcarbamate à activité herbicide ou au moins un acétanilide à activité herbicide.

8. Utilisation de la combinaison de substances actives selon la revendication 5 dans la lutte sélective contre les mauvaises herbes dans les cultures de végétaux utiles.

9. L'acétal de N-acylpipéridone de formule:

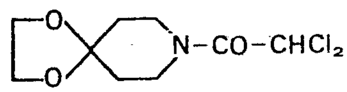

10. L'acétal de N-acylpipéridone de formule: